# EUROPÄISCHE PATENTSCHRIFT

(11) **EP 0 384 371 B1**
(45) Veröffentlichungstag und Bekanntmachung des Hinweises auf die Patenterteilung: **21.12.1994**
(21) Anmeldenummer: 90103187.2
(22) Anmeldetag: 20.02.1990
(51) Int. Cl.: C09K 3/30

(54) **Druckgaspackung und Treibmittel für Aerosole**
Aerosol container and propellant
Emballage pour aérosol et propulseur

(30) Priorität: 24.02.1989 DE 3905726
(43) Veröffentlichungstag der Anmeldung: 29.08.1990
(73) Patentinhaber: SOLVAY (Société Anonyme), B-1050 Bruxelles (BE)
(72) Erfinder: Heiskel, Elmar, Dr., D-6072 Dreieich (DE); Schmieder, Wilfried, Dr., D-6238 Hofheim am Taunus (DE)
(74) Vertreter: Jacques, Philippe

(56) Entgegenhaltungen:
- DE-A- 1 542 076
- GB-A- 1 298 263
- GB-A- 2 125 426
- DERWENT FILE SUPPLIER JAPS, Patent Office of Japan, Tokyo, JP; & JP-A-62 093 211

## Beschreibung

Seit Jahrzehnten werden Aerosol-Druckgaspackungen, kurz Aerosole genannt, unter Verwendung druckverflüssigter Gase (in einigen Fällen auch komprimierter Gase) als Treibmittel produziert und für mannigfache Zwecke verwendet. Als druckverflüssigte Gas (im folgenden auch als "Flüssiggase" bezeichnet) werden im wesentlichen die brennbaren Gase Propan/Butan (P/B) und Dimethylether (DME), sowie die unbrennbaren Fluorchlorkohlenwasserstoffe (FCKW), vornehmlich die Typen 12 (Dichlordifluormethan) und 114 (Dichlortetrafluorethan) verwendet.

Die brennbaren Flüssiggase bringen ein gewisses Sicherheitsrisiko mit sich. Aus diesem Grunde wurden in der Vergangenheit die nicht brennbaren und toxikologisch unbedenklichen FCKW bevorzugt eingesetzt. Die ebenfalls nicht brennbaren komprimierten Gase wie CO₂ oder N₂ sind nur in speziellen Fällen brauchbar, da sie in der Druckgas-Packung keinen vom Entleerungsgrad unabhängigen konstanten Druck aufrechterhalten können.

Seit Aufstellung der Ozon-Theorie (Abbau des Ozons durch FCKW und andere chlorhaltige organische Verbindungen) sucht man als Treibmittel geeignete Flüssiggase, die weder brennbar, noch in der Lage sind, Ozon abzubauen und außerdem nicht gesundheitsschädlich sind.

Unter Verwendung druckverflüssigter Gase können in ihren anwendungstechnischen Eigenschaften sehr unterschiedliche Druckgas-Packungen hergestellt werden. Druckgaspackungen bestehen immer aus einem Druckbehälter, vorzugsweise aus Metall oder Glas, der mit einer Ventilkonstruktion zur Entnahme des Inhaltes der Packung versehen ist, und dem im Behälter befindlichen Füllgut. Das Füllgut kann vielfältiger Natur sein.

Im einfachsten Fall besteht es nur aus einem singulären druckverflüssigten Gas, welches aus Behältern ohne Steigrohr bei Betätigung des Ventils aus der Gasphase, also in gasförmigem Zustand, ins Freie gelangt, wobei es einen druckluftähnlichen (Puster-)Effekt verursacht. Solche Produkte werden zur Entstaubung von z. B. Glasflächen, wie Kameralinsen, verwendet.

In den weitaus meisten Fällen besteht der Behälter-Inhalt jedoch aus einem sogenannten Füllprodukt (vielfach auch Wirkstofflösung genannt), welches versprüht werden soll, und einem Triebmittel in Form eines druckverflüssigten Gases oder Gasgemisches, welches in der Flüssigphase mit dem flüssigen Füllprodukt in jedem Verhältnis mischbar ist, also nur eine einzige flüssige Phase bildet, über der sich eine Gasphase ausbildet. Beispiele für solche (echten) Aerosol-Produkte, die als Nebel aus der Packung versprüht werden, sind z. B. Insektizidspray, Raumspray, Deodorantspray. Die haben relativ hohe Treibmittelanteile (> 50%). Voraussetzung für ihre Funktionsfähigkeit ist lückenlose Mischbarkeit des Lösemittels des Füllprodukts mit dem verflüssigten Treibgas(-Gemisch) sowie die Verwendung eines Ventils mit Steigrohr, das bis zum Boden des Behälters reicht.

Eine andere Art von Aerosolprodukten stellen die Schaumaerosole dar, bei denen die Flüssiganteile des Füllprodukts (z. B. Wasser) mit dem druckverflüssigten Treibgas wegen Fehlens der Mischbarkeit keine homogene Flüssigphase bilden, sondern zwei getrennte flüssige Phasen. Durch Schütteln des Behälters entsteht in Gegenwart eines Tensids aus den beiden flüssigen Phasen eine Emulsion (normalerweise eine O/W-, also "Öl in Wasser"-Emulsion). Bei deren Austritt aus dem Behälter durch ein Ventil mit "Schaumkopf" geht die Emulsion wegen des schlagartigen Verdampfens der Ölphase (also der Flüssiggaströpfchen) in einen Schaum über, dessen Volumen 200- bis 300mal größer ist als das der Emulsion. Solche Schaumaerosole werden vornehmlich im kosmetischen Bereich eingesetzt (z. B. Shampoo, Duschschaum, Sonnenschutzschaum). Voraussetzung für ihre Herstellung ist, daß sich Füllprodukt und Flüssiggas nicht homogen mischen, das bedeutet eine sehr geringe Löslichkeit der Flüssiggasphase in der flüssigen Füllproduktphase; dies ist im allgemeinen gegeben, wenn das Füllprodukt wäßrig ist, also "wasserbasierend".

Aus DE-OS 1 542 076 sind bereits Treibmittelmischungen bekannt, die ein relativ wasserlösliches Treibmittel aus der Gruppe der verflüssigten halogenierten Kohlenwasserstoffe und ein relativ wasserunlösliches Treibmittel aus derselben Gruppe enthalten. 2H-Heptafluorpropan wird dabei als Beispiel für ein relativ wasserunlösliches Treibmittel genannt.

GB-A-1 298 263 offenbart den Einsatz von Fluorkohlenwasserstoffen, Kohlenwasserstoffen und Dimethylether oder deren kombinationen, im mit ihnen befüllte Mikrokapseln beim Erwärmen zum Platzen zu bringen.

Ein Gegenstand der Erfindung ist ein Treibmittel für Aerosole, bestehend aus druckverflüssigtem 2-Hydro-heptafluorpropan (F 227) im Gemisch mit druckverflüssigtem Propan und/oder n-Butan und/oder i-Butan und/oder Dimethylether und/oder 1,1-Difluorethan.

Eine Aerosol-Druckgaspackung enthält neben den genannten Gemischen (als Treibmittel) ein Füllprodukt, wobei das Treibmittel und das Füllprodukt eine einzige oder auch zwei Flüssigphasen bilden können.

Ein besonders wichtiger Fall sind Aerosol-Druckgaspackungen, bei denen das Füllprodukt als Flüssiganteil Wasser enthält; dabei ist das Füllprodukt in allgemeinen eine wäßrige Lösung, manchmal aber auch eine Suspension. In diesem Fall liegt stets eine zweite flüssige (nämlich wäßrige) Phase neben dem druckverflüssigten Treibmittel-Gemisch vor.
Die druckverflüssigten Mischungskomponenten Propan, n-Butan, i-Butan, Dimethylether und 1,1-Difluorethan haben sämtlich eine Dichte unter 1 g/cm³, während F 227 eine Dichte über 1 g/cm³ hat. Daher kann man aus F 227 und den genannten Mischungskomponenten Gemische mit einer Dichte von etwa 1 g/cm³ herstellen. Solche Gemische haben daher die gleiche oder eine sehr ähnliche Dichte wie eine wäßrige Phase und sind daher sehr gut geeignet als Treibmittel für wasserbasierende Aerosole. In entsprechenden Druckgaspackungen entstehen bereits beim ersten Schütteln stabile O/W-Emulsionen, die sich auch bei längerem Stehen nicht mehr in zwei kontinuierliche Phasen trennen und daher vor erneuter Anwendung nicht nochmals geschüttelt werden müssen.

Besonders geeignete Treibmittelmischungen für wasserbasierende Aerosole sind die folgenden:
1. ein Gemisch aus F 227 und Propan/n-Butan
   (Massenverhältnis 15 : 85) im Massenverhältnis von 65 : 35 bis 85 : 15, vorzugsweise von 70 : 30 bis 80 : 20, insbesondere etwa 75 : 25.
2. ein Gemisch aus F 227 und i-Butan im Massenverhältnis von 65 : 35 bis 85 : 15, vorzugsweise 70 : 30 bis 80 : 20, insbesondere etwa 74 : 26.
3. ein Gemisch aus F 227 und Propan/i-Butan
   (Massenverhältnis 65 : 35) im Massenverhältis von 70 : 30 bis 90 : 10, vorzugsweise von 75 : 25 bis 85 : 15, insbesondere etwa 80 : 20.
4. ein Gemisch aus F 227 und 1,1-Difluorethan (F 152 a) im Massenverhältnis von 35 : 65 bis 45 : 55, insbesondere etwa 40 : 60
Vorzugsweise verwendet man eine der Treibmittelmischungen 1 bis 3.

### Beispiele

Es wurden folgende Flüssiggas-Treibmittel-Gemische I bis V hergestellt:

| Komponenten | I **(Masse- %)** | II **(Masse-%)** | III **(Masse-%)** | IV **(Masse-%)** | V **(Masse-%)** |
|---|---|---|---|---|---|
| F 227 | 76,0 | 65,0 | 40,0 | 74,0 | 80,0 |
| Propan | 3,6 | - | - | - | 13,0 |
| n-Butan | 20,4 | - | - | - | - |
| i-Butan | - | - | - | 26,0 | 7,0 |
| F 152 a | - | - | 60,0 | - | - |
| DME | - | 35,0 | - | - | - |
| Summe | 100,0 | 100,0 | 100,0 | 100,0 | 100,0 |

| Dichte (kg/1) | | | | | |
|---|---|---|---|---|---|
| 20°C | 1,0 | 1,0 | 1,06 | 1,0 | 1,05 |
| 50°C | 0,9 | 0,9 | 0,97 | 0,9 | 0,96 |

| Druck (bar) | | | | | |
|---|---|---|---|---|---|
| 20°C | 3,6 | 4,7 | 5,3 | 3,5 | 5,3 |
| 50°C | 8,2 | 10,7 | 11,4 | 8,0 | 11,5 |

### Beispiel 1 (Rasierschäume)

In Aerosol-Glasflaschen mit handelsüblichem Aerosol-Ventil und Schaumkopf wurden folgende Aerosol-Abfüllungen hergestellt:

### Füllprodukt:

9,0 Masse-% Stearinsäure
1,0 Masse-% Polyglykol 400
1,0 Masse-% Polyglykol 1500
2,0 Masse-% Laurinsäuremonoethanolamid
3,0 Masse-% Glyzerin
4,0 Masse-% Triethanolamin, techn.
80,0 Masse-% Wasser dest.

Das Füllprodukt wurde im Verhältnis
90 Masse-% Füllprodukt
10 Masse-% Treibmittelgemisch
mit den Treibmittelgemischen I, III, IV, bzw. V abgefüllt. In allen 4 Fällen entstand nach dem Schütteln eine lagerstabile Emulsion, die auch nach mehrmonatigem Stehen sich nicht mehr in 2 flüssige kontinuierliche Phasen trennte. Bei Niederdrücken des Schaumkopfes entstand in jedem Fall ein feinporiger, stabiler Schaum, dessen Eigenschaften allen an einen Rasierschaum zu stellenden Anforderungen genügten. Alle Schäume, auch die mit den Treibmittelgemischen I, IV und V, konnten nicht entzündet werden, im Gegensatz zu handelsüblichen Rasierschaum-Aerosolen, die mit Propan/Butan abgefüllt waren.

### Beispiel 2 (Duscheschäume)

Wie in Beispiel 1 wurden Versuchsabfüllungen von Aerosol-Duscheschäumen nach folgendem Rezept hergestellt:

### Füllprodukt:

3,0 Masse-% Isopropylmyristat
2,0 Masse-% 1,2-Propylenglykol
60,0 Masse-% Na-Alkylpolyglykolethersulfat
10,0 Masse-% Palmkernfettsäuresarkosid
25,0 Masse-% Wasser dest.

### Abfüllverhältnis:

90 Masse-% Füllprodukt
10 Masse-% Treibmittelgemisch
Als Treibmittelphase wurden wie in Beispiel 1 die Gemische I, III, IV und V eingesetzt.

Die erhaltenen Ergebnisse waren denen im Beispiel 1 analog. Auch diese Schäume erwiesen sich als nicht entflammbar.

### Beispiel 3 (Körperdeodorant-Spray)

In Aerosol-Glasflaschen wurden die folgenden Versuchsabfüllungen hergestellt:

### Füllprodukt:

| | |
|---|---|
| 96,1 Masse-% | Ethanol (99,8 %ig) |
| 0,5 Masse-% | 5-Chlor-2-(2,4-dichlorphenoxy)-phenol |
| 1,0 Masse-% | Isopropylmyristat |
| 0,4 Masse-% | Parfümöl |
| 2,0 Masse-% | 1,2 Propylenglykol |
| 100,0 Masse-% | |

### Abfüllverhältnis:

30,0 Masse-% Füllprodukt
70,0 Masse-% Treibmittel
Als Treibmittel wurde einmal reines F 227 und einmal Treibmittelgemisch II eingesetzt. Beide Abfüllungen hatten eine sehr gute Sprühcharakteristik und waren lagerstabil bei -5°C sowie bei +40°C. Geruchlich übertraf die Abfüllung mit dem Gemisch II noch die durchaus einwandfreie Abfüllung mit reinem 2-Hydro-heptafluorpropan. Die Abfüllung mit reinem F 227 als Treibmittel ist nach EG-Recht nicht mit "BRENNBAR" zu kennzeichnen.

### Beispiel 4 (Parfümspray)

Es wurde ein Parfümspray nach folgendem Rezept hergestellt:

### Füllprodukt:

| | |
|---|---|
| 5,0 Masse-% | Parfümöl |
| 95,0 Masse-% | Ethanol (96 %ig) |
| 100,0 Masse-% | |

### Aerosol-Abfüllung:

40,0 Masse-% Füllprodukt
60,0 Masse-% Treibmittel = F 227
Das Versuchsmuster zeigte ausgezeichnete Sprayeigenschaften und Lagerstabilität. Es wurde keine Beeinträchtigung der Duftnote durch das Treibmittel festgestellt. Produkte, die nach diesem Rezept hergestellt werden, sind laut EG-Richtlinie nicht mit "BRENNBAR" zu kennzeichnen.

## Patentansprüche (Patentansprüche für folgende(n) Vertragsstaat(en): AT, BE, CH, DE, DK, FR, GB, IT, LI, LU, NL, SE)

1. Treibmittel für Aerosole, bestehend aus druckverflüssigtem 2-Hydro-heptafluorpropan im Gemisch mit druckverflüssigtem Propan und/oder n-Butan und/oder i-Butan und/oder Dimethylether und/oder 1,1-Difluorethan.

2. Verwendung von druckverflüssigtem 2-Hydro-heptafluorpropan im Gemisch mit druckverflüssigtem Propan und/oder n-Butan und/oder i-Butan und/oder Dimethylether und/oder 1,1-Difluorethan als Treibmittel für Aerosole.

3. Druckgaspackung, enthaltend druckverflüssigtes 2-Hydro-heptafluorpropan im Gemisch mit druckverflüssigtem Propan und/oder n-Butan und/oder i-Butan und/oder Dimethylether und/oder 1,1-Difluorethan.

4. Aerosol-Druckgaspackung, enthaltend ein Treibmittel und ein Füllprodukt, die ein oder zwei Flüssigphasen bilden können, wobei das Treibmittel aus druckverflüssigtem 2-Hydro-heptafluorpropan im Gemisch mit druckverflüssigtem Propan und/oder n-Butan und/oder i-Butan und/oder Dimethylether und/oder 1,1-Difluorethan besteht.

5. Aerosol-Druckgaspackung nach Anspruch 4, dadurch gekennzeichnet, daß das Füllprodukt als Flüssiganteil Wasser enthält und eine zweite flüssige Phase bildet.

6. Aerosol-Druckgaspackung nach Anspruch 5, dadurch gekennzeichnet, daß als Treibmittel ein Gemisch aus druckverflüssigtem 2-Hydro-heptafluorpropan und Propan und/oder n-Butan und/oder i-Butan und/oder Dimethylether und/oder 1,1-Difluorethan eingesetzt wird, welches etwa die gleiche Dichte wie das Wasser enthaltende Füllprodukt hat.

7. Aerosol-Druckgaspackung nach Anspruch 5, dadurch gekennzeichnet, daß als Treibmittel ein Gemisch aus 2-Hydro-heptafluorpropan und Propan/n-Butan (Massenverhältnis 15 : 85) im Massenverhältnis von 65 : 35 bis 85 : 15 eingesetzt wird.

8. Aerosol-Druckgaspackung nach Anspruch 7, dadurch gekennzeichnet, daß das Massenverhältnis 70 : 30 bis 80 : 20 beträgt.

9. Aerosol-Druckgaspackung nach Anspruch 5, dadurch gekennzeichnet, daß als Treibmittel ein Gemisch aus 2-Hydro-heptafluorpropan und i-Butan im Massenverhältnis von 65 : 35 bis 85 : 15 eingesetzt wird.

10. Aerosol Druckgaspackung nach Anspruch 9, dadurch gekennzeichnet, daß das Massenverhältnis 70 : 30 bis 80 : 20 beträgt.

11. Aerosol-Druckgaspackung nach Anspruch 5, dadurch gekennzeichnet, daß als Treibmittel ein Gemisch aus 2-Hydro-heptafluorpropan und Propan/i-Butan (Massenverhältnis 65 : 35) im Massenverhältnis von 70 : 30 bis 90 : 10 eingesetzt wird.

12. Aerosol-Druckgaspackung nach Anspruch 11, dadurch gekennzeichnet, daß das Massenverhältnis 75 : 25 bis 85 : 15 beträgt.

13. Aerosol-Druckgaspackung nach Anspruch 5, dadurch gekennzeichnet, daß als Treibmittel ein Gemisch aus 2-Hydro-heptafluorpropan und 1,1-Difluorethan im Massenverhältnis von 30 : 70 bis 50 : 50 eingesetzt wird.

14. Aerosol-Druckgaspackung nach Anspruch 13, dadurch gekennzeichnet, daß das Masseverhältnis 35 : 65 bis 45 : 55 beträgt.

## Patentansprüche (Patentansprüche für folgende(n) Vertragsstaat(en): ES, GR)

1. Verwendung von druckverflüssigtem 2-Hydro-heptafluorpropan im Gemisch mit druckverflüssigtem Propan und/oder n-Butan und/oder i-Butan und/oder Dimethylether und/oder 1,1-Difluorethan als Treibmittel für Aerosole.

2. Verfahren zur Herstellung einer eine Treibmittelmischung enthaltenden Druckgaspackung, dadurch gekennzeichnet, daß man druckverflüssigtes 2-Hydro-heptafluorpropan und druckverflüssigtes Propan und/oder n-Butan und/oder i-Butan und/oder Dimethylether und/oder 1,1-Difluorethan in den Behälter der Druckgaspackung einfüllt, wobei das Gemisch der Treibmittel vor oder nach dem Einfüllen gebildet werden kann.

3. Verfahren zur Herstellung einer Aerosol-Druckgaspackung, enthaltend eine Treibmittelmischung und ein Füllprodukt, die ein oder zwei Flüssigphasen bilden können, dadurch gekennzeichnet, daß man druckverflüssigtes 2-Hydro-heptafluorpropan und druckverflüssigtes Propan und/oder n-Butan und/oder i-Butan und/oder Dimethylether und/oder 1,1-Difluorethan sowie das Füllprodukt in den Behälter der Druckgaspackung einfüllt, wobei das Gemisch der Treibmittel vor oder nach dem Einfüllen gebildet werden kann.

4. Verfahren nach Anspruch 3, dadurch gekennzeichnet, daß das Füllprodukt als Flüssiganteil Wasser enthält und eine zweite flüssige Phase bildet.

5. Verfahren nach Anspruch 4, dadurch gekennzeichnet, daß als Treibmittel ein Gemisch aus druckverflüssigtem 2-Hydro-heptafluorpropan und Propan und/oder n-Butan und/oder i-Butan und/oder Dimethylether und/oder 1,1-Difluorethan eingesetzt wird, welches etwa die gleiche Dichte wie das Wasser enthaltende Füllprodukt hat.

6. Verfahren nach Anspruch 4, dadurch gekennzeichnet, daß als Treibmittel ein Gemisch aus 2-Hydro-heptafluorpropan und Propan/n-Butan (Massenverhältnis 15:85) im Massenverhältnis von 65:35 bis 85:15 eingesetzt wird.

7. Verfahren nach Anspruch 6, dadurch gekennzeichnet, daß das Massenverhältnis 70:30 bis 80:20 beträgt.

8. Verfahren nach Anspruch 4, dadurch gekennzeichnet, daß als Treibmittel ein Gemisch aus 2-Hydro-heptafluorpropan und Propan/n-Butan im Massenverhältnis von 65:35 bis 85:15 eingesetzt wird.

9. Verfahren nach Anspruch 8, dadurch gekennzeichnet, daß das Massenverhältnis 70:30 bis 80:20 beträgt.

10. Verfahren nach Anspruch 4, dadurch gekennzeichnet, daß als Treibmittel ein Gemisch aus 2-Hydro-heptafluorpropan und Propan/i-Butan (Massenverhältnis 65:35) im Massenverhältnis von 70:30 bis 90:10 eingesetzt wird.

11. Verfahren nach Anspruch 10, dadurch gekennzeichnet, daß das Massenverhältnis 75:25 bis 85:15 beträgt.

12. Verfahren nach Anspruch 4, dadurch gekennzeichnet, daß als Treibmittel ein Gemisch aus 2-Hydro-heptafluorpropan und 1,1-Difluorethan im Massenverhältnis von 30:70 bis 50:50 eingesetzt wird.

13. Verfahren nach Anspruch 12, dadurch gekennzeichnet, daß das Massenverhältnis 35:65 bis 45:55 beträgt.

## Claims (Claims for the following Contracting State(s): AT, BE, CH, DE, DK, FR, GB, IT, LI, LU, NL, SE)

1. A propellant for aerosols, comprising pressure-liquefied 2-hydro-heptafluoropropane in a mixture with pressure-liquefied propane and/or n-butane and/or i-butane and/or dimethyl ether and/or 1,1-difluoroethane.

2. The use of pressure-liquefied 2-hydro-heptafluoropropane in a mixture with pressure-liquefied propane and/or n-butane and/or i-butane and/or dimethyl ether and/or 1,1-difluoroethane as propellant for aerosols.

3. A pressurized-gas pack, containing pressure-liquefied 2-hydro-heptafluoropropane in a mixture with pressure-liquefied propane and/or n-butane and/or i-butane and/or dimethyl ether and/or 1,1-difluoroethane.

4. An aerosol pressurized-gas pack, containing a propellant and a filling product, which can form one or two liquid phases, the propellant comprising pressure-liquefied 2-hydro-heptafluoropropane in a mixture with pressure-liquefied propane and/or n-butane and/or i-butane and/or dimethyl ether and/or 1,1-difluoroethane.

5. The aerosol pressurized-gas pack as claimed in claim 4, wherein the filling product contains water as the liquid component and forms a second liquid phase.

6. The aerosol pressurized-gas pack as claimed in claim 5, wherein the propellant used is a mixture of pressure-liquefied 2-hydro-heptafluoropropane and propane and/or n-butane and/or i-butane and/or dimethyl ether and/or 1,1-difluoroethane, which mixture has approximately the same density as the water-containing filling product.

7. The aerosol pressurized-gas pack as claimed in claim 5, wherein the propellant used is a mixture of 2-hydro-heptafluoropropane and propane/n-butane (ratio by mass 15 : 85) in the ratio by mass of 65 : 35 to 85 : 15.

8. The aerosol pressurized-gas pack as claimed in claim 7, wherein the ratio by mass is 70 : 30 to 80 : 20.

9. The aerosol pressurized-gas pack as claimed in claim 5, wherein the propellant used is a mixture of 2-hydro-heptafluoropropane and i-butane in the ratio by mass of 65 : 35 to 85 : 15.

10. The aerosol pressurized-gas pack as claimed in claim 9, wherein the ratio by mass is 70 : 30 to 80 : 20.

11. The aerosol pressurized-gas pack as claimed in claim 5, wherein the propellant used is a mixture of 2-hydro-heptafluoropropane and propane/i-butane (ratio by mass 65 : 35) in the ratio by mass of 70 : 30 to 90 : 10.

12. The aerosol pressurized-gas pack as claimed in claim 11, wherein the ratio by mass is 75 : 25 to 85 : 15.

13. The aerosol pressurized-gas pack as claimed in claim 5, wherein the propellant used is a mixture of 2-hydro-heptafluoropropane and 1,1-difluoroethane in the ratio by mass of 30 : 70 to 50 : 50.

14. The aerosol pressurized-gas pack as claimed in claim 13, wherein the ratio by mass is 35 : 65 to 45 : 55.

## Claims (Claims for the following Contracting State(s): ES, GR)

1. The use of pressure-liquefied 2-hydro-heptafluoropropane in a mixture with pressure-liquefied propane and/or n-butane and/or i-butane and/or dimethyl ether and/or 1,1-difluoroethane as propellant for aerosols.

2. A process for producing a pressurized-gas pack containing a propellant mixture, which comprises introducing pressure-liquefied 2-hydro-heptafluoropropane and pressure-liquefied propane and/or n-butane and/or i-butane and/or dimethyl ether and/or 1,1-difluoroethane into the container of the pressurized-gas pack, it being possible to form the mixture of propellants before or after the filling operation.

3. A process for producing an aerosol pressurized-gas pack, containing a propellant mixture and a filling product, which can form one or two liquid phases, which comprises introducing pressure-liquefied 2-hydro-heptafluoropropane and pressure-liquefied propane and/or n-butane and/or i-butane and/or dimethyl ether and/or 1,1-difluoroethane and the filling product into the container of the pressurized-gas pack, it being possible to form the mixture of propellants before or after the filling operation.

4. The process as claimed in claim 3, wherein the filling product contains water as the liquid component and forms a second liquid phase.

5. The process as claimed in claim 4, wherein the propellant used is a mixture of pressure-liquefied 2-hydro-heptafluoropropane and propane and/or n-butane and/or i-butane and/or dimethyl ether and/or 1,1-difluoroethane, which mixture has approximately the same density as the water-containing filling product.

6. The process as claimed in claim 4, wherein the propellant used is a mixture of 2-hydro-heptafluoropropane and propane/n-butane (ratio by mass 15 : 85) in the ratio by mass of 65 : 35 to 85 : 15.

7. The process as claimed in claim 6, wherein the ratio by mass is 70 : 30 to 80 : 20.

8. The process as claimed in claim 4, wherein the propellant used is a mixture of 2-hydro-heptafluoropropane and propane/n-butane in the ratio by mass of 65 : 35 to 85 : 15.

9. The process as claimed in claim 8, wherein the ratio by mass is 70 : 30 to 80 : 20.

10. The process as claimed in claim 4, wherein the propellant used is a mixture of 2-hydro-heptafluoropropane and propane/i-butane (ratio by mass 65 : 35) in the ratio by mass of 70 : 30 to 90 : 10.

11. The process as claimed in claim 10, wherein the ratio by mass is 75 : 25 to 85 : 15.

12. The process as claimed in claim 4, wherein the propellant used is a mixture of 2-hydro-heptafluoropropane and 1,1-difluoroethane in the ratio by mass of 30 : 70 to 50 : 50.

13. The process as claimed in claim 12, wherein the ratio by mass is 35 : 65 to 45 : 55.

## Revendications (Revendications pour l'(les) Etat(s) contractant(s) suivant(s): AT, BE, CH, DE, DK, FR, GB, IT, LI, LU, NL, SE)

1. Propulseur pour aérosols, constitué de 2-hydro-heptafluoropropane liquéfié sous pression, en mélange avec du propane et/ou du n-butane et/ou de l'isobutane et/ou de l'oxyde de diméthyle et/ou du 1,1-difluoroéthane liquéfiés sous pression.

2. Utilisation du 2-hydro-heptafluoropropane liquéfié sous pression en mélange avec du propane et/ou du n-butane et/ou de l'isobutane et/ou de l'oxyde de diméthyle et/ou du 1,1-difluoroéthane liquéfiés sous pression en tant que propulseurs pour aérosols.

3. Emballage à gaz sous pression contenant du 2-hydro-heptafluoropropane liquéfié sous pression, en mélange avec du propane et/ou du n-butane et/ou de l'isobutane et/ou de l'oxyde de diméthyle et/ou du 1,1-difluoroéthane liquéfiés sous pression.

4. Emballage aérosol à gaz sous pression contenant un propulseur et un produit de remplissage, qui peuvent former une ou deux phases liquides, le propulseur étant constitué de 2-hydro-heptafluoropropane liquéfié sous pression en mélange avec du propane et/ou du n-butane et/ou de l'isobutane et/ou de l'oxyde de diméthyle et/ou du 1,1-difluoroéthane liquéfiés sous pression.

5. Emballage aérosol à gaz sous pression selon la revendication 4, caractérisé en ce que le produit de remplissage contient de l'eau en tant que fraction liquide et forme une deuxième phase liquide.

6. Emballage aérosol à gaz sous pression selon la revendication 5, caractérisé en ce qu'on utilise comme propulseur un mélange de 2-hydro-heptafluoropropane liquéfié sous pression et de propane et/ou de n-butane et/ou d'isobutane et/ou d'oxyde de diméthyle et/ou de 1,1-difluoroéthane liquéfiés sous pression, lequel mélange a approximativement la même masse volumique que le produit de remplissage contenant de l'eau.

7. Emballage aérosol à gaz sous pression selon la revendication 5, caractérisé en ce qu'on utilise comme propulseur un mélange de 2-hydro-heptafluoropropane et de propane/n-butane (rapport en masse 15:85) selon un rapport en masse de 65:35 à 85:15.

8. Emballage aérosol à gaz sous pression selon la revendication 7, caractérisé en ce que le rapport en masse est de 70:30 à 80:20.

9. Emballage aérosol à gaz sous pression selon la revendication 5, caractérisé en ce qu'on utilise comme propulseur un mélange de 2-hydro-heptafluropropane et d'isobutane selon un rapport en masse de 65:35 à 85:15.

10. Emballage aérosol à gaz sous pression selon la revendication 9, caractérisé en ce que le rapport en masse est de 70:30 à 80:20.

11. Emballage aérosol à gaz sous pression selon la revendication 5, caractérisé en ce qu'on utilise comme propulseur un mélange de 2-hydro-heptafluoropropane et de propane/isobutane (rapport en masse 65:35) selon un rapport en masse de 70:30 à 90:10.

12. Emballage aérosol à gaz sous pression selon la revendication 11, caractérisé en ce que le rapport en masse est de 75:25 à 85:15.

13. Emballage aérosol à gaz sous pression selon la revendication 5, caractérisé en ce qu'on utilise comme propulseur un mélange de 2-hydro-heptafluoropropane et de 1,1-difluoroéthane selon un rapport en masse de 30:70 à 50:50.

14. Emballage aérosol à gaz sous pression selon la revendication 13, caractérisé en ce que le rapport en masse est de 35:65 à 45:55.

## Revendications (Revendications pour l'(les) Etat(s) contractant(s) suivant(s): ES, GR)

1. Utilisation du 2-hydro-heptafluoropropane liquéfié sous pression en mélange avec du propane et/ou du n-butane et/ou de l'isobutane et/ou de l'oxyde de diméthyle et/ou du 1,1-difluoroéthane liquéfiés sous pression en tant que propulseurs pour aérosols.

2. Procédé pour fabriquer un emballage à gaz sous pression contenant un mélange de propulseurs, caractérisé en ce qu'on introduit dans le récipient de l'emballage à gaz sous pression du 2-hydro-heptafluoropropane liquéfié sous pression et du propane et/ou du n-butane et/ou de l'isobutane et/ou de l'oxyde de diméthyle et/ou du 1,1-difluoroéthane liquéfiés sous pression, le mélange des propulseurs pouvant être constitué avant ou après le remplissage.

3. Procédé pour fabriquer un emballage aérosol à gaz sous pression, contenant un mélange de propulseurs et un produit de remplissage, qui peuvent former une ou deux phases liquides, caractérisé en ce qu'on introduit dans le récipient de l'emballage à gaz sous pression du 2-hydro-heptafluoropropane liquéfié sous pression et du propane et/ou du n-butane et/ou de l'isobutane et/ou de l'oxyde de diméthyle et/ou du 1,1-difluoroéthane liquéfiés sous pression, ainsi que le produit de remplissage, le mélange des propulseurs pouvant être constitué avant ou après le remplissage.

4. Procédé selon la revendication 3, caractérisé en ce que le produit de remplissage contient de l'eau en tant que fraction liquide et forme une deuxième phase liquide.

5. Procédé selon la revendication 4, caractérisé en ce qu'on utilise comme propulseur un mélange de 2-hydro-heptafluoropropane liquéfié sous pression et de propane et/ou de n-butane et/ou d'isobutane et/ou d'oxyde de diméthyle et/ou de 1,1-difluoroéthane liquéfiés sous pression, lequel mélange a approximativement la même masse volumique que le produit de remplissage contenant de l'eau.

6. Procédé selon la revendication 4, caractérisé en ce qu'on utilise comme propulseur un mélange de 2-hydro-heptafluoropropane et de propane/n-butane (rapport en masse 15:85) selon un rapport en masse de 65:35 à 85:15.

7. Procédé selon la revendication 6, caractérisé en ce que le rapport en masse est de 70:30 à 80:20.

8. Procédé selon la revendication 4, caractérisé en ce qu'on utilise comme propulseur un mélange de 2-hydro-heptafluropropane et d'isobutane selon un rapport en masse de 65:35 à 85:15.

9. Procédé selon la revendication 8, caractérisé en ce que le rapport en masse est de 70:30 à 80:20.

10. Procédé selon la revendication 4, caractérisé en ce qu'on utilise comme propulseur un mélange de 2-hydro-heptafluoropropane et de propane/isobutane (rapport en masse 65:35) selon un rapport en masse de 70:30 à 90:10.

11. Procédé selon la revendication 10, caractérisé en ce que le rapport en masse est de 75:25 à 85:15.

12. Procédé selon la revendication 4, caractérisé en ce qu'on utilise comme propulseur un mélange de 2-hydro-heptafluoropropane et de 1,1-difluoroéthane selon un rapport en masse de 30:70 à 50:50.

13. Procédé selon la revendication 12, caractérisé en ce que le rapport en masse est de 35:65 à 45:55.
